# EUROPEAN PATENT SPECIFICATION

(11) **EP 2 449 092 B1**
(45) Date of publication and mention of the grant of the patent: **12.08.2015**
(21) Application number: 10730012.1
(22) Date of filing: 30.06.2010
(51) Int. Cl.: C12N 1/22, C12P 7/10, C12P 19/02, C12P 19/14

(54) **BIOMASS HYDROLYSIS PROCESS**
BIOMASSE-HYDROLYSEVERFAHREN
PROCÉDÉ D'HYDROLYSE D'UNE BIOMASSE

(30) Priority: 30.06.2009 US 221689 P
(43) Date of publication of application: 09.05.2012
(73) Proprietor: Novozymes A/S, 2880 Bagsvaerd (DK); Novozymes North America, Inc., Franklinton, North Carolina 27525 (US)
(72) Inventor: REN, Haiyu, Beijing 100085 (CN); HUANG, Hong, Zhi, Beijing 100088 (CN); WANG, Yun, Beijing 100083 (CN); HIGGINS, Don, Franklinton North Carolina 27525 (US)
(74) Representative: Rasmussen, Preben
(86) International application number: PCT/US2010/040518
(87) International publication number: WO 2011/002832

(56) References cited:
- WO-A1-01/60752
- WO-A1-94/21785
- WO-A1-2008/134259
- WO-A1-2009/030713
- WO-A2-2010/011957
- KUMAR RAJEEV ET AL: "Effect of enzyme supplementation at moderate cellulase loadings on initial glucose and xylose release from corn stover solids pretreated by leading technologies." BIOTECHNOLOGY AND BIOENGINEERING, vol. 102, no. 2, 1 February 2009 (2009-02-01), pages 457-467, XP002600812 ISSN: 1097-0290
- KUMAR R ET AL: "Effect of xylanase supplementation of cellulase on digestion of corn stover solids prepared by leading pretreatment technologies" BIORESOURCE TECHNOLOGY, vol. 100, no. 18, 21 April 2009 (2009-04-21), pages 4203-4213, XP026130071 ISSN: 0960-8524 [retrieved on 2009-04-21]
- KUMAR RAJEEV ET AL: "Effects of Cellulase and Xylanase Enzymes on the Deconstruction of Solids from Pretreatment of Poplar by Leading Technologies" BIOTECHNOLOGY PROGRESS, vol. 25, no. 2, Sp. Iss. SI, March 2009 (2009-03), pages 302-314, XP002600813 ISSN: 8756-7938
- PALMQVIST EVA ET AL: "Fermentation of lignocellulosic hydrolysates. I: Inhibition and detoxification" BIORESOURCE TECHNOLOGY, vol. 74, no. 1, 1 August 2000 (2000-08-01) , pages 17-24, XP002584226 ISSN: 0960-8524

### SEQUENCE LISTING

<110> Novozymes A/S
   Novozymes North America, Inc.
<120> Biomass hydrolysis process
<130> 11580-WO-PCT
<160> 1
<170> PatentIn version 3.5
<210> 1
   <211> 406
   <212> PRT
   <213> Aspergillus aculeatus
<400> 1
   Met Val Gly Leu Leu Ser Ile Thr Ala Ala Leu Ala Ala Thr Val Leu
   1               5                   10                  15
   Pro Asn Ile Val Ser Ala Val Gly Leu Asp Gln Ala Ala Val Ala Lys
               20                  25                  30
   Gly Leu Gln Tyr Phe Gly Thr Ala Thr Asp Asn Pro Glu Leu Thr Asp
           35                  40                  45
   Ile Pro Tyr Val Thr Gln Leu Asn Asn Thr Ala Asp Phe Gly Gln Ile
       50                  55                  60
   Thr Pro Gly Asn Ser Met Lys Trp Asp Ala Thr Glu Pro Ser Gln Gly
   65                  70                  75                      80
   Thr Phe Thr Phe Thr Lys Gly Asp Val Ile Ala Asp Leu Ala Glu Gly
                   85                  90                  95
   Asn Gly Gln Tyr Leu Arg Cys His Thr Leu Val Trp Tyr Asn Gln Leu
               100                 105                 110
   Pro Ser Trp Val Thr Ser Gly Thr Trp Thr Asn Ala Thr Leu Thr Ala
           115                 120                 125
   Ala Leu Lys Asn His Ile Thr Asn Val Val Ser His Tyr Lys Gly Lys
       130                 135                 140
   Cys Leu His Trp Asp Val Val Asn Glu Ala Leu Asn Asp Asp Gly Thr
   145                 150                 155                     160
   Tyr Arg Thr Asn Ile Phe Tyr Thr Thr Ile Gly Glu Ala Tyr Ile Pro
                   165                 170                 175
   Ile Ala Phe Ala Ala Ala Ala Ala Ala Asp Pro Asp Ala Lys Leu Phe
               180                 185                 190
   Tyr Asn Asp Tyr Asn Leu Glu Tyr Gly Gly Ala Lys Ala Ala Ser Ala
           195                 200                 205
   Arg Ala Ile Val Gln Leu Val Lys Asn Ala Gly Ala Lys Ile Asp Gly
       210                 215                 220
   Val Gly Leu Gln Ala His Phe Ser Val Gly Thr Val Pro Ser Thr Ser
   225                 230                 235                 240
   Ser Leu Val Ser Val Leu Gln Ser Phe Thr Ala Leu Gly Val Glu Val
                   245                 250                 255
   Ala Tyr Thr Glu Ala Asp Val Arg Ile Leu Leu Pro Thr Thr Ala Thr
               260                 265                 270
   Thr Leu Ala Gln Gln Ser Ser Asp Phe Gln Ala Leu Val Gln Ser Cys
           275                 280                 285
   Val Gln Thr Thr Gly Cys Val Gly Phe Thr Ile Trp Asp Trp Thr Asp
       290                 295                 300
   Lys Tyr Ser Trp Val Pro Ser Thr Phe Ser Gly Tyr Gly Ala Ala Leu
   305                 310                 315                 320
   Pro Trp Asp Glu Asn Leu Val Lys Lys Pro Ala Tyr Asn Gly Leu Leu
                   325                 330                 335
   Ala Gly Met Gly Val Thr Val Thr Thr Thr Thr Thr Thr Thr Thr Ala
               340                 345                 350
   Thr Ala Thr Gly Lys Thr Thr Thr Thr Thr Thr Gly Ala Thr Ser Thr
           355                 360                 365
   Gly Thr Thr Ala Ala His Trp Gly Gln Cys Gly Gly Leu Asn Trp Ser
       370                 375                 380
   Gly Pro Thr Ala Cys Ala Thr Gly Tyr Thr Cys Thr Tyr Val Asn Asp
   385                 390                 395                 400
   Tyr Tyr Ser Gln Cys Leu
                   405

## Description

### TECHNICAL FIELD

The present invention relates to processes comprising recycling of washing solution used in the pretreatment of lignocellulose-containing materials.

### BACKGROUND OF THE INVENTION

Due to the limited reserves of fossil fuels and worries about emission of greenhouse gasses there is an increasing focus on using renewable energy sources, *e.g.,* fermentation products, such as bioethanol. Production of ethanol from lignocellulose-containing material is known in the art and conventionally includes pretreatment, hydrolysis, and fermentation of the lignocellulose-containing material. Pretreatment results in the release of degradation products from the lignocellulose-containing material that may irreversibly bind and inhibit enzymes added during hydrolysis and fermentation.

The concentration of these inhibitors in the pretreated lignocellulose-containing material can be reduced by washing before hydrolysis. However, the inhibitors often have low solubility and recycling of the wash water is only possible to a very limited extend due to build-up of the inhibitors. Washing therefore requires huge amounts of water.

Consequently, there is a need for providing improved methods for processing pretreated lignocellulose-containing material into substrates suitable for hydrolysis and fermentation.

WO 2009/030713 discloses a process for converting a lignocellulose-containing material into a hydrolyzate comprising the steps of: (a) subjecting a lignocellulose-containing material to a pretreatment, (b) washing the pretreated lignocellulose-containing material in a washing solution, (c) separating off the used washing solution to obtain a pretreated and washed lignocellulose-containing material, (d) subjecting the pretreated and washed lignocellulose-containing material to a treatment resulting in at least partial hydrolysis of the cellulose and/or hemicellulose to obtain a hydrolyzate, wherein the used washing solution of step (b) is treated to remove an enzyme inhibitor and/or an inhibitor of a fermenting organism before being recycled to step (b), preferably wherein the used washing solution of step (b) is treated by contacting it with a resin column before being recycled to step (b).

### SUMMARY OF THE INVENTION

The present invention relates to processes comprising removal from and/or inactivation of inhibitors from pretreated lignocellulose-containing material by washing, and recycling of the used washing solution. The used washing solution is regenerated by removal and/or inactivation of an enzyme inhibitor, thereby improving the efficacy of the hydrolysis step. The invention also relates to processes of producing a hydrolyzate and a fermentation product from lignocellulose-containing material.

Accordingly, the invention relates to a process for converting a lignocellulose-containing material into a hydrolyzate comprising the steps of: (a) subjecting a lignocellulose-containing material to a pretreatment, (b) washing the pretreated lignocellulose-containing material in a washing solution, (c) separating off the used washing solution to obtain a pretreated and washed lignocellulose-containing material, (d) subjecting the pretreated and washed lignocellulose-containing material to a treatment resulting in at least partial hydrolysis of the cellulose and/or hemicellulose to obtain a hydrolyzate, and (e) treating the used washing solution of step (c) with a xylanase before being recycled to step (b).

The advantages of the processes include a reduction of water consumption as the washing solution can be recycled, the soluble sugars *(e.g.,* xylose, oligossachrides) produced from pretreatment can be concentrated in the washing solution, and increased efficiency of the hydrolysis.

### DETAILED DESCRIPTION OF THE INVENTION

### Lignocellulose-Containing Material

The term "lignocellulose-containing materials" used herein refer to a material primarily consisting of cellulose, hemicellulose, and lignin. Lignocellulose-containing materials are often referred to as "biomass".

The structure of lignocellulose is not directly accessible to enzymatic hydrolysis. Therefore, the lignocellulose has to be pretreated, *e.g.,* by acid hydrolysis under adequate conditions of pressure, humidity and temperature, in order to break the lignin seal and disrupt the crystalline structure of cellulose. This causes solubilization and saccharification of the hemicellulose fraction. The cellulose fraction can then be hydrolyzed, *e.g.,* enzymatically by cellulase enzymes, to convert the carbohydrate polymers into mono- and oligosaccharides, which may be fermented into a desired fermentation product, such as ethanol. Optionally the fermentation product is recovered, *e.g.,* by distillation.

Any lignocellulose-containing material is contemplated according to the present invention. The lignocellulose-containing material may be any material containing lignocellulose. In a preferred embodiment the lignocellulose-containing material contains at least 30 wt. %, preferably at least 50 wt. %, more preferably at least 70 wt. %, even more preferably at least 90 wt. % lignocellulose. It is to be understood that the lignocellulose-containing material may also comprise other constituents such as cellulosic material, including cellulose and hemicellulose, and may also comprise other constituents such as proteinaceous material, starch, sugars, such as fermentable sugars and/or un-fermentable sugars.

Lignocellulose-containing material is generally found, for example, in the stems, leaves, hulls, husks, and cobs of plants or leaves, branches, and wood of trees. Lignocellulose-containing material can be, but is not limited to, herbaceous material, agricultural residues, forestry residues, municipal solid wastes, waste paper, and pulp and paper mill residues. It is understood herein that lignocellulose-containing material may be in the form of plant cell wall material containing lignin, cellulose, and hemicellulose in a mixed matrix.

The lignocellulose-containing material may comprise corn stover, hard wood, such as poplar and birch, soft wood such as pine wood, switch grass, cereal straw and/or husks, such as straw from rice, wheat, barley rye etc., municipal solid waste (MSW), industrial organic waste, office paper, wood chips, bagasse, paper or pulp processing waste or mixtures thereof.

In a preferred embodiment the cellulose-containing material is corn stover. In another preferred aspect, the cellulose-containing material is corn fibre.

### Pretreatment

The lignocellulose-containing material may be pretreated in any suitable way. Pretreatment is carried out before hydrolysis and/or fermentation. The goal of pretreatment is to separate and/or release cellulose, hemicellulose and/or lignin and this way improve the rate of hydrolysis. Pretreatment methods such as wet-oxidation and alkaline pretreatment targets lignin, while dilute acid and auto-hydrolysis targets hemicellulose. Steam explosion is an example of a pretreatment that targets cellulose.

According to the invention pretreatment step (a) may be a conventional pretreatment step using techniques well known in the art. In a preferred embodiment pretreatment takes place in an aqueous slurry. The lignocellulose-containing material may during pretreatment be present in an amount between 10-80 wt. %, preferably between 20-70 wt. %, especially between 30-60 wt. %, such as around 50 wt. %.

The pretreatment is carried out prior to the hydrolysis and/or fermentation.

The term "chemical treatment" refers to any chemical pretreatment which promotes the separation and/or release of cellulose, hemicellulose and/or lignin. Examples of suitable chemical pretreatments include treatment with, for example, dilute acid, lime, alkaline, organic solvent, ammonia, sulfur dioxide, carbon dioxide. Further, wet oxidation and pH-controlled hydrothermolysis are also considered chemical pretreatment.

In a preferred embodiment the chemical pretreatment is acid treatment, more preferably, a continuous dilute and/or mild acid treatment, such as, treatment with sulfuric acid, or another organic acid, such as acetic acid, citric acid, tartaric acid, succinic acid, hydrogen chloride or mixtures thereof. Other acids may also be used. Mild acid treatment means that the treatment pH lies in the range from 1-5, preferably pH 1-3. In a specific embodiment the acid concentration is in the range from 0.1 to 2.0 wt % acid, preferably sulphuric acid. The acid may be contacted with the lignocellulose-containing material and the mixture may be held at a temperature in the range of 160-220°C, such as 165-195°C, for periods ranging, *e.g.,* 1-60 minutes, such as 2-30 minutes or 3-12 minutes. Addition of strong acids, such as sulphuric acid, may be applied to remove hemicellulose. This enhances the digestibility of cellulose.

Other techniques are also contemplated. Cellulose solvent treatment has been shown to convert about 90% of cellulose to glucose. It has also been shown that enzymatic hydrolysis could be greatly enhanced when the lignocellulose structure is disrupted. Alkaline H₂O₂, ozone, organosolv (uses Lewis acids, FeCl₃, (Al)₂SO₄ in aqueous alcohols), glycerol, dioxane, phenol, or ethylene glycol are among solvents known to disrupt cellulose structure and promote hydrolysis (Mosier et al., 2005, Bioresource Technology 96: 673-686).

Alkaline chemical pretreatment with base, e.g., NaOH, Na₂CO₃ and/or ammonia or the like, is also contemplated according to the invention. Pretreatment methods using ammonia is described in, *e.g.,* WO 2006/110891, WO 2006/110899, WO 2006/110900, and WO 2006/110901.

Wet oxidation techniques involve use of oxidizing agents, such as sulphite based oxidizing agents or the like. Examples of solvent pretreatments include treatment with DMSO (Dimethyl Sulfoxide) or the like. Chemical pretreatment is generally carried out for 1 to 60 minutes, such as from 5 to 30 minutes, but may be carried out for shorter or longer periods of time dependent on the material to be pretreated.

Other examples of suitable pretreatment methods are described by Schell et al., 2003, Appl. Biochem, and Biotechn. 105-108: 69-85; Mosier et al., 2005, Bioresource Technology 96: 673-686; Ahring et al. in WO 2006/032282 and WO 2001/060752, Foody et al. in WO 2006/034590; and Ballesteros et al. in US publication no. 2002/0164730.

The term "mechanical pretreatment" refers to any mechanical (or physical) treatment which promotes the separation and/or release of cellulose, hemicellulose and/or lignin from lignocellulose-containing material. For example, mechanical pretreatment includes various types of milling, irradiation, steaming/steam explosion, wet oxidation, and other hydrothermal treatments.

Mechanical pretreatment includes comminution (mechanical reduction of the size). Comminution includes dry milling, wet milling and vibratory ball milling. Mechanical pretreatment may involve high pressure and/or high temperature (steam explosion). In an embodiment of the invention high pressure means pressure in the range from 300 to 600 psi, preferably 400 to 500 psi, such as around 450 psi. In an embodiment of the invention high temperature means temperatures in the range from about 100 to 300°C, preferably from about 140 to 235°C. In a preferred embodiment mechanical pretreatment is a batch-process, steam gun hydrolyzer system which uses high pressure and high temperature as defined above. A Sunds Hydrolyzer (available from Sunds Defibrator AB (Sweden) may be used for this.

In a preferred embodiment both chemical and mechanical pretreatments are carried out. For instance, the pretreatment step may involve dilute or mild acid treatment and high temperature and/or pressure treatment. The chemical and mechanical pretreatment may be carried out sequentially or simultaneously, as desired.

Accordingly, in a preferred embodiment, the lignocellulose-containing material is subjected to both chemical and mechanical pretreatment to promote the separation and/or release of cellulose, hemicellulose and/or lignin.

In a preferred embodiment a mechanical pretreatment is carried out before a stream explosion pretreatment.

In a preferred embodiment the pretreatment is carried out as a dilute and/or mild acid steam explosion step. In another preferred embodiment pretreatment is carried out as an ammonia fiber explosion step (or AFEX pretreatment step).

As used in the present invention the term "biological pretreatment" refers to any biological pretreatment which promotes the separation and/or release of cellulose, hemicellulose, and/or lignin from the lignocellulose-containing material. Biological pretreatment techniques can involve applying lignin-solubilizing microorganisms (see, for example, Hsu, T.-A., 1996, Pretreatment of biomass, in Handbook on Bioethanol: Production and Utilization, Wyman, C. E., ed., Taylor & Francis, Washington, DC, 179-212; Ghosh, P., and Singh, A., 1993, Physicochemical and biological treatments for enzymatic/microbial conversion of lignocellulosic biomass, Adv. Appl. Microbiol. 39: 295-333; McMillan, J. D., 1994, Pretreating lignocellulosic biomass: a review, in Enzymatic Conversion of Biomass for Fuels Production, Himmel, M. E., Baker, J. O., and Overend, R. P., eds., ACS Symposium Series 566, American Chemical Society, Washington, DC, chapter 15; Gong, C. S., Cao, N. J., Du, J., and Tsao, G. T., 1999, Ethanol production from renewable resources, in Advances in Biochemical Engineering/Biotechnology, Scheper, T., ed., Springer-Verlag Berlin Heidelberg, Germany, 65: 207-241; Olsson, L., and Hahn-Hagerdal, B., 1996, Fermentation of lignocellulosic hydrolysates for ethanol production, Enz. Microb. Tech. 18: 312-331; and Vallander, L., and Eriksson, K.-E. L., 1990, Production of ethanol from lignocellulosic materials: State of the art, Adv. Biochem. Eng./Biotechnol 42: 63-95).

### Washing of Pretreated Lignocellulose-Containing Material

When lignocellulose-containing material is pretreated, degradation products that are inhibitory to enzymes are produced. These compounds severely decrease the hydrolysis rate.

Washing of pretreated lignocellulose-containing material in order to remove inhibitors of enzymes will improve the enzymatic hydrolysis.

The inhibitors are lignocellulose degradation products including lignin degradation products, cellulose degradation products and hemicellulose degradation products. The lignin degradation products may be phenolic in nature. The hemicellulose degradation products include furans from sugars (such as hexoses and/or pentoses), including mannose, galactose, rhamanose, arabinose and xylose, including oligosaccharides. The compounds inhibitory to enzymes are believed to include xylooligosaccharides (XOOs) or complexes of XOO and soluble lignin, present in the PCS liquor. According to the present invention soluble compounds inhibitory to enzymes are removed from the pretreated lignocellulose-containing material by washing with a washing solution. The washing solution is preferably an aqueous washing solution. The washing solution may be a substantially pure solution of water, or water with a significant amount of additives, e.g., such as a detergent and/or an organic solvent to improve the extraction and/or solubility of the compounds inhibitory to enzymes. A suitable organic solvent is ethanol or methanol.

The washing solution is preferably applied at a temperature of 5 to 70°C, preferably of 10 to 65°C, and more preferably 20 to 60°C, *e.g.,* at a temperature from 30 to 55°C.

The washing step ends when the used washing solution is separated from the washed PCS. The separation of the used washing solution may be achieved by any suitable method including but not limited to draining, filtration, centrifugation and pressing.

According to the present invention the used washing solution is treated to remove and/or inactivate an enzyme inhibitor before being recycled to the washing step. The treatment regenerates the used washing solution in order to create a usable washing solution. The treatment comprises contacting the used washing solution with a xylanase, and more preferably an immobilized xylanase. Without being bound by theory, it is believed that the effect of the process of the present invention is caused by the xylanase activity on soluble components, in particular on xylooligosaccharides (XOOs) or complexes of XOO and soluble lignin, present in the PCS liquor.

In an embodiment the used washing solution is also treated by contacting it with a resin or a combination of resins. The combination of resins may be applied as a mixture of two or more resins and/or it may be two or more resins applied one after the other. The resin may be a resin with a polar, a weakly polar or non-polar functional group. The resin may be a strongly acidic cation exchanger, a weakly acidic cation exchanger, a strongly basic anion exchanger, or a weakly basic anion exchanger. Preferred functional groups include -SO₃, -COOH, -N+(CH₃)₃, -N(CH₃)₂ and -NH₂. The resin may also be a charcoal resin. In an embodiment the used washing solution is treated by contacting it with activated charcoal.

In an embodiment the used washing solution is passed through a column comprising the resin and/or activated charcoal.

### Hydrolysis

Before and/or simultaneously with fermentation the pretreated and washed lignocellulose-containing material is hydrolyzed to break down cellulose and hemicellulose into sugars and/or oligosaccharides.

The dry solids content during hydrolysis may be in the range from 5-50 wt. %, preferably 10-40 wt. %, more preferably 20-35 wt. %. Hydrolysis may in a preferred embodiment be carried out as a fed batch process where the pretreated lignocellulose-containing material (substrate) is fed gradually to an, *e.g.,* enzyme containing hydrolysis solution. The pretreated lignocellulose-containing material may be supplied to the enzyme containing hydrolysis solution either in one or more distinct batches, as one or more distinct continuous flows or as a combination of one or more distinct batches and one or more distinct continuous flows.

Hydrolysis is carried out enzymatically. According to the invention, the pretreated lignocellulose-containing material may be hydrolyzed by one or more hydrolases (class EC 3 according to Enzyme Nomenclature), preferably one or more carbohydrases selected from the group consisting of cellulase, hemicellulase, alpha-amylase, glucoamylase, esterase, such as lipase, or protease. Alpha-amylase and/or glucoamylase may be present during hydrolysis and/or fermentation as the lignocellulose-containing material may include some starch.

The enzyme(s) used for hydrolysis is(are) capable of directly or indirectly converting the washed pretreated lignocellulose-containing material into fermentable sugars which can be fermented into a desired fermentation product, such as ethanol.

Suitable enzymes are described in the "Enzymes"-section below.

Hemicellulose polymers can be broken down by hemicellulases and/or acid hydrolysis to release its five and six carbon sugar components. The six carbon sugars (hexoses), such as glucose, galactose, arabinose, and mannose, can readily be fermented to, *e.g.,* ethanol, acetone, butanol, glycerol, citric acid, fumaric acid etc. by suitable fermenting organisms including yeast.

In a preferred embodiment the pretreated lignocellulose-containing material is hydrolyzed using a hemicellulase, preferably a xylanase, esterase, cellobiase, or combination thereof.

Enzymatic treatment may be carried out in a suitable aqueous environment under conditions which can readily be determined by one skilled in the art. In a preferred embodiment hydrolysis is carried out at suitable, preferably optimal conditions for the enzyme(s) in question.

Suitable process time, temperature and pH conditions can readily be determined by one skilled in the art. Preferably, hydrolysis is carried out at a temperature between 25°C and 70°C, preferably between 40°C and 60°C, especially around 50°C. The process is preferably carried out at a pH in the range from 3-8, preferably pH 4-6, especially around pH 5. Preferably, hydrolysis is carried out for between 12 and 144 hours, preferable 16 to 120 hours, more preferably between 24 and 96 hours, such as between 32 and 72 hours.

According to the invention hydrolysis in step (c) and fermentation in step (d) may be carried out simultaneously (SHHF process) or sequentially (SHF process).

### Fermentation

According to the invention the pretreated (and hydrolyzed) lignocellulose-containing material is fermented by at least one fermenting organism capable of fermenting fermentable sugars, such as glucose, xylose, mannose, and galactose directly or indirectly into a desired fermentation product.

The fermentation is preferably ongoing for between 8 to 96 hours, preferably 12 to 72, more preferable from 24 to 48 hours. In an embodiment the fermentation is carried out at a temperature between 20 to 40°C, preferably 26 to 34°C, in particular around 32°C. In an embodiment the pH is from pH 3 to 6, preferably around pH 4 to 5.

Preferred for ethanol fermentation is yeast of the species *Saccharomyces cerevisiae,* preferably strains which are resistant towards high levels of ethanol, *i.e.,* up to, *e.g.,* about 10, 12 or 15 vol. % ethanol or more, such as 20 vol. % ethanol.

Contemplated according to the invention is simultaneous hydrolysis and fermentation (SHF). In an embodiment there is no separate holding stage for the hydrolysis, meaning that the hydrolysing enzyme(s) and the fermenting organism are added together. When the fermentation is performed simultaneous with hydrolysis the temperature is preferably between 26°C and 35°C, more preferably between 30°C and 34°C, such as around 32°C. A temperature program comprising at least two holding stages at different temperatures may be applied according to the invention.

During washing of the pretreated lignocellulose-containing material dissolved sugars may accumulate in the recycled aqueous washing solution. The dissolved sugars will comprise C5 sugars from the degradation of the hemicellulose, such as xylose. These sugars can be fermented with a suitable fermenting organism which is able to convert C5 sugars into a desired fermentation product. This C5 fermentation may be performed separately, or the dissolved sugars accumulated in the recycled aqueous washing solution may be added to the hydrolyzed lignocellulose-containing material for a combined C6 and C5 fermentation. Such a fermentation is preferably performed with at least one organism able to ferment both C6 (*e.g*., glucose) and C5 sugars (*e.g*., xylose). Alternatively, fermentation may be performed with at least two separate organisms, each optimized to utilizing either the C6 or the C5 sugars, either in one fermentation step under conditions allowing both organisms to ferment, or as at least two fermentation steps, each under conditions allowing one of the organisms to ferment,

The process of the invention may be performed as a batch, fed-batch or as a continuous process. Preferably the fermentation step is performed as a continuous fermentation.

### Recovery

Subsequent to fermentation the fermentation product may be separated from the fermentation broth. The broth may be distilled to extract the fermentation product or the fermentation product may be extracted from the fermentation broth by micro or membrane filtration techniques. Alternatively the fermentation product may be recovered by stripping. Recovery methods are well known in the art.

### Fermentation Products

The process of the invention may be used for producing any fermentation product. Especially contemplated fermentation products include alcohols (*e.g.,* ethanol, methanol, butanol); organic acids (*e.g*., citric acid, acetic acid, itaconic acid, lactic acid, gluconic acid); ketones (*e.g.,* acetone); amino acids *(e.g.,* glutamic acid); gases (*e.g.,* H₂ and CO₂); antibiotics (*e.g.,* penicillin and tetracycline); enzymes; vitamins (*e.g.,* riboflavin, B12, beta-carotene); and hormones.

Also contemplated products include consumable alcohol industry products, e.g., beer and wine; dairy industry products, *e.g.,* fermented dairy products; leather industry products and tobacco industry products. In a preferred embodiment the fermentation product is an alcohol, especially ethanol. The fermentation product, such as ethanol, obtained according to the invention, may preferably be fuel alcohol/ethanol. However, in the case of ethanol it may also be used as potable ethanol.

### Fermenting Organism

The term "fermenting organism" refers to any organism, including bacterial and fungal organisms, suitable for producing a desired fermentation product. Especially suitable fermenting organisms according to the invention are able to ferment, *i.e.,* convert, sugars, such as glucose, directly or indirectly into the desired fermentation product. Also suitable are fermenting organisms capable of converting C5 sugars such as xylose into a desired fermentation product. Examples of fermenting organisms include fungal organisms, especially yeast. Preferred yeast includes strains of *Saccharomyces* spp., in particular a strain of *Saccharomyces cerevisiae* or *Saccharomyces uvarum;* a strain of *Pichia,* preferably *Pichia stipitis,* such as *Pichia stipitis* CBS 5773; a strain of *Candida,* in particular a strain of *Candida utilis, Candida diddensii,* or *Candida boidinii.* Other contemplated yeast includes strains of *Zymomonas*; *Hansenula,* in particular *H. anomala; Klyveromyces,* in particular *K. fragilis*; and *Schizosaccharomyces,* in particular *S*. *pombe.*

Commercially available yeast includes, *e.g.,* ETHANOL RED™ yeast (available from Fermentis/Lesaffre, USA), FALI (available from Fleischmann's Yeast, USA), SUPERSTART and THERMOSACC™ fresh yeast (available from Ethanol Technology, WI, USA), BIOFERM AFT and XR (available from NABC - North American Bioproducts Corporation, GA, USA), GERT STRAND (available from Gert Strand AB, Sweden), and FERMIOL (available from DSM Specialties). ANQI YEAST (available from Anqi yeast (CHIFENG) CO., LTD, China).

### Enzymes

Even though not specifically mentioned in the context of a process of the invention, it is to be understood that the enzymes (as well as other compounds) are used in an "effective amount".

Cellulases: The term "cellulases" as used herein are understood as comprising the cellobiohydrolases (EC 3.2.1.91), *e.g.,* cellobiohydrolase I and cellobiohydrolase I as well as the endo-glucanases (EC 3.2.1.4) and beta-glucosidases (EC 3.2.1.21). Cellulases are applied in the hydrolysis step (d).

In order to be efficient, the digestion of cellulose and hemicellulose requires several types of enzymes acting cooperatively. At least three categories of enzymes are necessary to convert cellulose into fermentable sugars: endo-glucanases (EC 3.2.1.4) that cut the cellulose chains at random; cellobiohydrolases (EC 3.2.1.91) which cleave cellobiosyl units from the cellulose chain ends and beta-glucosidases (EC 3.2.1.21) that convert cellobiose and soluble cellodextrins into glucose. Among these three categories of enzymes involved in the biodegradation of cellulose, cellobiohydrolases are the key enzymes for the degradation of native crystalline cellulose. The term "cellobiohydrolase I" is defined herein as a cellulose 1,4-beta-cellobiosidase (also referred to as Exo-glucanase, Exo-cellobiohydrolase or 1,4-beta-cellobiohydrolase) activity, as defined in the enzyme class EC 3.2.1.91, which catalyzes the hydrolysis of 1,4-beta-D-glucosidic linkages in cellulose and cellotetraose, by the release of cellobiose from the non-reducing ends of the chains. The definition of the term "cellobiohydrolase II activity" is identical, except that cellobiohydrolase II attacks from the reducing ends of the chains.

Endoglucanases (EC No. 3.2.1.4) catalyze endo hydrolysis of 1,4- beta -D-glycosidic linkages in cellulose, cellulose derivatives (such as carboxy methyl cellulose and hydroxy ethyl cellulose), lichenin, beta-1,4 bonds in mixed beta-1,3 glucans such as cereal beta-D-glucans or xyloglucans and other plant material containing cellulosic parts. The authorized name is endo-1,4-beta-D-glucan 4-glucano hydrolase, but the abbreviated term endoglucanase is used in the present specification.

The cellulase activity may, in a preferred embodiment, be derived from a fungal source, such as a strain of the genus *Trichoderma,* preferably a strain of *Trichoderma reesei*; a strain of the genus *Humicola,* such as a strain of *Humicola insolens;* or a strain of *Chrysosporium,* preferably a strain of *Chrysosporium lucknowense.*

In a preferred embodiment the cellulase preparation comprising a polypeptide having cellulolytic enhancing activity (GH61A), preferably the one disclosed in WO 2005/074656. The cellulase preparation may further comprise a beta-glucosidase, such as the fusion protein disclosed in U.S. provisional application no. 60/832,511. In an embodiment the cellulase preparation also comprises a CBH II, preferably *Thielavia terrestris* cellobiohydrolase II CEL6A. In an embodiment the cellulase preparation also comprises a cellulase preparation derived from *Trichoderma reesei.* In a preferred embodiment the cellulase preparation is Cellulase preparation A used in Example 1 and disclosed in WO 2008/151079.

In an embodiment the cellulase is the commercially available product CELLUCLAST® 1.5L or CELLUZYME™ (Novozymes A/S, Denmark).

The cellulase may be dosed in the range from 0.1-100 FPU per gram dry solids (DS), preferably 0.5-50 FPU per gram DS, especially 1-20 FPU per gram DS.

The cellulase may be dosed in the range from 0.1-10000 mg enzyme protein (EP)/kg dry solids (DS), preferably 0.5-5000 mg EP/kg DS, especially 1-2500 mg EP/kg DS.

Hemicellulases: Hemicellulose can be broken down by hemicellulases and/or acid hydrolysis to release its five and six carbon sugar components.

Any hemicellulase suitable for use in hydrolyzing hemicellulose may be used. Preferred hemicellulases include xylanases, arabinofuranosidases, acetyl xylan esterases, ferulic acid esterases, glucuronidases, endo-galactanases, mannases, endo or exo arabinases, endo or exo galactanases, and mixtures of two or more thereof. Preferably, the hemicellulase for use in the present invention is an exo-acting hemicellulase, and more preferably, the hemicellulase is an exo-acting hemicellulase which has the ability to hydrolyze hemicellulose under acidic conditions of below pH 7, preferably pH 3-7. Examples of hemicellulase compositions suitable for use in the present invention include VISCOZYME™ and ULTRAFLO™ (available from Novozymes A/S, Denmark).

Xylanase (EC 3.2.1.8) for use in the present invention is preferably an endo-1,4-beta-xylanase, and preferably of Glycoside Hydrolase Family 10 or 11 (GH10 or GH11). The GH10 or GH11 are defined in Cantarel et al., 2008, Nucleic Acids Res. 37: D233-D238 and on the internet (cazy.org).

The xylanase may be of any origin including mammalian, plant or animal origin; however, it is preferred that the xylanase is of microbial origin. In particular the xylanase may be one derivable from a filamentous fungus or a yeast. Preferably the xylanase is derived from a filamentous fungus such as from *Aspergillus sp., Bacillus sp., Humicola sp., Myceliophotora sp., Poitrasia sp., Rhizomucor sp.* or *Trichoderma.* The xylanase is preferably a GH10 xylanase.

Most preferred is a xylanase derived from *Aspergillus aculeatus* and disclosed as xylanase II in WO 1994/021785 and as SEQ ID NO:1 herein. Also preferred are xylanases having at least 50% identity, at least 60% identity, at least 70% identity, at least 80% identity, at least 90% identity, at least 95% identity, or even at least 99% identity with the amino acid sequence shown in SEQ ID NO:1 herein.

The xylanase applied in the process of the present invention for treating the used washing solution is preferably an immobilized xylanase. If a non-immobilized xylanase is used it is added in amounts of 0.001-1.0 g/kg DS substrate, preferably in the amounts of 0.005-0.5 g/kg DS substrate, and most preferably from 0.05-0.10 g/kg DS substrate.

A xylanase may also be added in the hydrolysis step of the present invention in amounts of 0.001-1.0 g/kg DS substrate, preferably in the amounts of 0.005-0.5 g/kg DS substrate, and most preferably from 0.05-0.10 g/kg DS substrate.

Ferulic acid esterase (EC 3.1.1.73) catalyzes the hydrolysis of the 4-hydroxy-3-methoxycinnamoyl (feruloyl) group from an esterified sugar, which is usually arabinose in arabinoxylan. A suitable ferulic acid esterase may be obtained from a strain of a filamentous fungus (*e.g*., *Trichoderma, Meripilus, Humicola, Aspergillus, Fusarium)* or from a bacteria (*e.g., Bacillus*), such as from *Aspergillus niger, e.g.,* the FAEIII ferulic acid esterase described by Faulds et al. 1994, Microbiology, 140, pp. 779-787.

Arabinofuranosidase (EC 3.2.1.55) catalyzes the hydrolysis of terminal non-reducing alpha-L-arabinofuranoside residues in alpha -L-arabinosides.

Galactanase (EC 3.2.1.89), arabinogalactan endo-1,4-beta-galactosidase, catalyzes the endohydrolysis of 1,4-D-galactosidic linkages in arabinogalactans.

Pectinase (EC 3.2.1.15) catalyzes the hydrolysis of 1,4-alpha-D-galactosiduronic linkages in pectate and other galacturonans.

Xyloglucanase catalyzes the hydrolysis of xyloglucan.

The hemicellulase may be added in an amount effective to hydrolyze hemicellulose, such as, in amounts from about 0.001 to 0.5 wt. % of dry solids (DS), more preferably from about 0.05 to 0.5 wt. % of DS.

### MATERIALS & METHODS

### Enzymes:

A xylanase composition comprising the xylanase shown in SEQ ID NO:1.

Cellulase preparation A comprising cellulolytic enzymes derived from *Trichoderma reesei,* a polypeptide having cellulolytic enhancing activity (GH61A) disclosed in WO 2005/074656, and an *Aspergillus oryzae* beta-glucosidase (in the fusion protein disclosed in WO 2008/057637). Cellulase preparation A is disclosed in WO 2008/151079.

### Determination of identity

The relatedness between two amino acid sequences or between two nucleotide sequences is described by the parameter "identity".

The degree of identity between two amino acid sequences may be determined by the Clustal method (Higgins, 1989, CABIOS 5: 151-153) using the LASERGENE™ MEGALIGN™ software (DNASTAR, Inc., Madison, WI) with an identity table and the following multiple alignment parameters: Gap penalty of 10 and gap length penalty of 10. Pairwise alignment parameters are Ktuple=1, gap penalty=3, windows=5, and diagonals=5.

The degree of identity between two nucleotide sequences may be determined by the Wilbur-Lipman method (Wilbur and Lipman, 1983, Proceedings of the National Academy of Science USA 80: 726-730) using the LASERGENE™ MEGALIGN™ software (DNASTAR, Inc., Madison, WI) with an identity table and the following multiple alignment parameters: Gap penalty of 10 and gap length penalty of 10. Pairwise alignment parameters are Ktuple=3, gap penalty=3, and windows=20.

### EXAMPLES

### Example 1

Corn stover was pretreated using steam explosion at 195°C for 5.4 min. The pretreated corn stover (PCS) 250 g with a dry solids content (DS) of 53.10% was mixed with 1L aqueous washing solution (water to PCS weight ration 4:1) and incubated with stirring for 30 minutes at room temperature. The spent washing solution from the PCS was squeezed out through 8 layers of cheese cloth and filtered through 32 layers of cheese cloth. Washed PCS was collected and about 880 g spent washing solution were obtained. The spent washing solution was treated with 8.8 ml xylanase (176 mg enzyme protein, at 50°C for 90 minutes, followed by 90°C for 30 minutes for inactivation of enzyme. The spent washing solution of 880 g was mixed with 220 g unwashed PCS and incubated with stirring for 30 minutes at room temperature. The spent washing solution from the PCS was squeezed out through 8 layers of cheese cloth and filtered through 32 layers of cheese cloth. Washed PCS was collected and about 770 g spent washing solution was obtained. The spent washing solution was treated with 7.7 ml xylanase (154 mg enzyme protein, at 50°C for 90 minutes, followed by 90°C for 30 minutes for inactivation of enzyme. The spent washing solution of 770 g was mixed with 192.5 g unwashed PCS and incubated with stirring for 30 minutes at room temperature. The spent washing solution from the PCS was squeezed out through 8 layers of cheese cloth and filtered through 32 layers of cheese cloth. Washed PCS was collected. Three batches of washed PCS were obtained. The washed PCS were portioned into samples of initial DS of 6% and a weight of 20 g. Penicillin was added for controlling bacterial contamination. Cellulase composition A was added in a concentration of 6 mg EP/g cellulose and hydrolysis was performed for 72 hours at 50°C and pH 5.0.

For the control, the unwashed PCS was washed as the procedure above except the xylanase solution was substituted with water. Three batches of washed PCS were obtained.

Following hydrolysis the soluble C6 sugars was quantified by HPLC. The results are shown in Table 1.

| Table 1. Glucose equivalent conversion (%) of washed PCS determined by HPLC | | |
|---|---|---|
| Cycle No. | Control | Treated with xylanase |
| I | 59.87 | 59.24 |
| II | 50.61 | 58.32 |
| III | 45.39 | 53.37 |

Inactivation of the xylanase by the 30 minutes incubation at 90°C was performed in this example only to demonstrate that the effect of the xylanase was due activity towards a soluble substrate in the washing solution - and not activity towards the solid substrate of the PCS.

## Claims

1. A process for converting a lignocellulose-containing material into a hydrolyzate comprising the steps of:
a) subjecting a lignocellulose-containing material to a pretreatment,
b) washing the pretreated lignocellulose-containing material in a washing solution,
c) separating off the used washing solution to obtain a pretreated and washed lignocellulose-containing material,
d) subjecting the pretreated and washed lignocellulose-containing material to a treatment resulting in at least partial hydrolysis of the cellulose and/or hemicellulose to obtain a hydrolyzate, and
e) treating the used washing solution of step (c) with a xylanase before being recycled to step (b).

2. The process of claim 1, further comprising, contacting the hydrolyzate of step (d) with a fermenting organism to produce a fermentation product.

3. The process of claim 2, comprising simultaneous hydrolysis and fermentation.

4. The process of claim 2 or 3, further comprising recovery of the fermentation product.

5. The process of any of the preceding claims, wherein the xylanase is a GH10 xylanase.

6. The process of any of the preceding claims, wherein the xylanase is immobilized.

7. The process of any of the preceding claims, wherein the lignocellulose-containing material originates from materials selected from the group comprising: corn stover, corn fiber, hard wood, such as poplar and birch, soft wood, cereal straw, such as, wheat straw, switch grass, rice hulls, municipal solid waste, industrial organic waste, office paper, or mixtures thereof.

8. The process of any of the preceding claims, wherein the lignocellulose-containing material in step (a) is chemically and/or mechanically pretreated.

9. The process of any of the preceding claims, wherein the lignocellulose-containing material in step (a) is chemically pretreated using acid.

10. The process of any of the preceding claims, wherein the pretreatment in step (a) is acid pretreated is carried out using an organic acid, preferably sulphuric acid, acetic acid, citric acid, tartaric acid, succinic acid, and/or mixtures thereof.

11. The process of any of the preceding claims, wherein the lignocellulose-containing material in step (a) is acid pretreated with from 0.1 to 2.0 wt. % acid, preferably sulfuric acid.

12. The process of any of the preceding claims, wherein the lignocellulose-containing material in step (a) is mechanically pretreated at a high temperature and/or a high pressure.

13. The process of any of the preceding claims, wherein the lignocellulose-containing material in step (a) is pretreated by steam explosion, dilute acid steam explosion and/or wet oxidation.

14. The process of any of the preceding claims, wherein the washing solution of step (b) comprises water, organic solvent or a mixture of water and an organic solvent

15. The process of any of the preceding claims, wherein dissolved sugars accumulating in the recycled aqueous washing solution (i) is lead to the fermentation step (d) and fermented with a C5 fermenting organism, or (ii) is separated out, and fermented with a C5 fermenting organism.

## Patentansprüche

1. Verfahren zum Umsetzen eines Lignocellulose-enthaltenden Materials in ein Hydrolysat umfassend die Schritte von:
a) Unterziehen eines Lignocellulose-enthaltenden Materials einer Vorbehandlung,
b) Waschen des vorbehandelten Lignocellulose-enthaltenden Materials in einer Waschlösung,
c) Abtrennen von der verwendeten Waschlösung, um ein vorbehandeltes und gewaschenes Lignocellulose-enthaltendes Material zu erhalten,
d) Unterziehen des vorbehandelten und gewaschenen Lignocellulose-enthaltenden Materials einer Behandlung resultierend in mindestens teilweiser Hydrolyse der Cellulose und/oder Hemicellulose, um ein Hydrolysat zu erhalten und
e) Behandeln der verwendeten Waschlösung von Schritt c) mit einer Xylanase bevor sie an Schritt b) recycelt wird.

2. Verfahren nach Anspruch 1, des Weiteren umfassend Inkontaktbringen des Hydrolysats von Schritt d) mit einem fermentierenden Organismus, um ein Fermentationsprodukt herzustellen.

3. Verfahren nach Anspruch 2, umfassend simultane Hydrolyse und Fermentation.

4. Verfahren nach Anspruch 2 oder 3, des Weiteren umfassend Gewinnen des Fermentationsprodukts.

5. Verfahren nach einem beliebigen der vorhergehenden Ansprüche, wobei die Xylanase eine GH10-Xylanase ist.

6. Verfahren nach einem beliebigen der vorhergehenden Ansprüche, wobei die Xylanase immobilisiert ist.

7. Verfahren nach einem beliebigen der vorhergehenden Ansprüche, wobei das Lignocellulose-enthaltende Material aus Materialien stammt ausgewählt aus der Gruppe umfassend: Maiserntereste, Maisfaser, Hartholz, wie Pappel und Birke, Weichholz, Getreidestroh, wie, Weizenstroh, Rutenhirse (switch grass), Reishülsen, kommunaler Festabfall, industrieller organischer Abfall, Büropapier, oder Mischungen davon.

8. Verfahren nach einem beliebigen der vorhergehenden Ansprüche, wobei das Lignocellulose-enthaltende Material in Schritt a) chemisch und/oder mechanisch vorbehandelt wird.

9. Verfahren nach einem beliebigen der vorhergehenden Ansprüche, wobei das Lignocellulose-enthaltende Material in Schritt a) unter Verwendung von Säure chemisch vorbehandelt wird.

10. Verfahren nach einem beliebigen der vorhergehenden Ansprüche, wobei die Behandlung in Schritt a) säure-vorbehandelt wird, durchgeführt unter Verwendung einer organischen Säure, vorzugsweise Schwefelsäure, Essigsäure, Zitronensäure, Weinsäure, Bernsteinsäure und/oder Mischungen davon.

11. Verfahren nach einem beliebigen der vorhergehenden Ansprüche, wobei das Lignocellulose-enthaltende Material in Schritt a) säure-vorbehandelt wird mit von 0,1 bis 2,0 Gew.-% Säure, vorzugsweise Schwefelsäure.

12. Verfahren nach einem beliebigen der vorhergehenden Ansprüche, wobei das Lignocellulose-enthaltende Material in Schritt a) bei einer hohen Temperatur und/oder einem hohen Druck mechanisch vorbehandelt wird.

13. Verfahren nach einem beliebigen der vorhergehenden Ansprüche, wobei das Lignocellulose-enthaltende Material in Schritt a) durch Dampfexplosion, verdünnte Säure-Dampfexplosion und/oder nasse Oxidation vorbehandelt wird.

14. Verfahren nach einem beliebigen der vorhergehenden Ansprüche, wobei die Waschlösung von Schritt b) Wasser, organisches Lösemittel oder eine Mischung von Wasser und einem organischen Lösemittel umfasst.

15. Verfahren nach einem beliebigen der vorhergehenden Ansprüche, wobei gelöste Zucker, welche in der recycelten wässrigen Waschlösung akkumulieren, (i) zu dem Fermentationsschritt d) geführt und mit einem C5-fermentierenden Organismus fermentiert werden, oder (ii) abgetrennt, und mit einem C5-fermentierenden Organismus fermentiert werden.

## Revendications

1. Procédé de conversion d'une substance contenant de la lignocellulose en un hydrolysat comprenant les étapes de :
a) soumission d'une substance contenant de la lignocellulose à un prétraitement,
b) lavage de la substance contenant de la lignocellulose prétraitée dans une solution de lavage,
c) séparation de la solution de lavage utilisée pour obtenir une substance contenant de la lignocellulose prétraitée et lavée,
d) soumission de la substance contenant de la lignocellulose prétraitée et lavée à un traitement entraînant une hydrolyse au moins partielle de la cellulose et/ou de l'hémicellulose pour obtenir un hydrolysat, et
e) traitement de la solution de lavage utilisée de l'étape (c) avec une xylanase avant son recyclage vers l'étape (b).

2. Procédé selon la revendication 1, comprenant en outre, la mise en contact de l'hydrolysat de l'étape (d) avec un organisme de fermentation pour produire un produit de fermentation.

3. Procédé selon la revendication 2, comprenant l'hydrolyse et la fermentation simultanées.

4. Procédé selon la revendication 2 ou 3, comprenant en outre la récupération du produit de fermentation.

5. Procédé selon l'une quelconque des revendications précédentes, dans lequel la xylanase est une xylanase GH10.

6. Procédé selon l'une quelconque des revendications précédentes, dans lequel la xylanase est immobilisée.

7. Procédé selon l'une quelconque des revendications précédentes, dans lequel la substance contenant de la lignocellulose provient de substances choisies dans le groupe constitué par la canne de maïs, la fibre de maïs, les bois durs tels que le peuplier et le bouleau, les bois tendres, la paille de céréales telle que la paille de blé, le panic raide, les balles de riz, les déchets solides municipaux, les déchets organiques industriels, le papier de bureau ou des mélanges de ceux-ci.

8. Procédé selon l'une quelconque des revendications précédentes, dans lequel la substance contenant de la lignocellulose de l'étape (a) est prétraitée chimiquement et/ou mécaniquement.

9. Procédé selon l'une quelconque des revendications précédentes, dans lequel la substance contenant de la lignocellulose de l'étape (a) est prétraitée chimiquement en utilisant un acide.

10. Procédé selon l'une quelconque des revendications précédentes, dans lequel le prétraitement de l'étape (a) est un prétraitement acide réalisé en utilisant un acide organique, de préférence l'acide sulfurique, l'acide acétique, l'acide citrique, l'acide tartrique, l'acide succinique et/ou des mélanges de ceux-ci.

11. Procédé selon l'une quelconque des revendications précédentes, dans lequel la substance contenant de la lignocellulose de l'étape (a) est prétraitée à l'acide avec de 0,1 à 2,0 % en poids d'acide, de préférence d'acide sulfurique.

12. Procédé selon l'une quelconque des revendications précédentes, dans lequel la substance contenant de la lignocellulose de l'étape (a) est prétraitée mécaniquement à une température élevée et/ou une pression élevée.

13. Procédé selon l'une quelconque des revendications précédentes, dans lequel la substance contenant de la lignocellulose de l'étape (a) est prétraitée par explosion de vapeur, explosion de vapeur d'acide dilué et/ou oxydation humide.

14. Procédé selon l'une quelconque des revendications précédentes, dans lequel la solution de lavage de l'étape (b) comprend de l'eau, un solvant organique ou un mélange d'eau et d'un solvant organique.

15. Procédé selon l'une quelconque des revendications précédentes, dans lequel les sucres dissous s'accumulant dans la solution de lavage aqueuse recyclée (i) sont envoyés vers l'étape de fermentation (d) et fermentés avec un organisme de fermentation en C5, ou (ii) sont séparés et fermentés avec un organisme de fermentation en C5.
